# EUROPÄISCHE PATENTSCHRIFT

(11) **EP 0 344 586 B1**
(45) Veröffentlichungstag und Bekanntmachung des Hinweises auf die Patenterteilung: **13.03.1996**
(21) Anmeldenummer: 89109312.2
(22) Anmeldetag: 24.05.1989
(51) Int. Cl.: A61K 31/71, A61K 31/52

(54) **TNF-Inhibitor enthaltendes Arzneimittel**
Medicine containing a TNF inhibitor
Médicament contenant un inhibiteur TNF

(30) Priorität: 27.05.1988 DE 3817955
(43) Veröffentlichungstag der Anmeldung: 06.12.1989
(73) Patentinhaber: SCHRINNER, Elmar J., D-65191 Wiesbaden (DE)
(72) Erfinder: Hänel, Heinz, Dr., D-6370 Oberursel (DE); Schrinner, Elmar, Dr., D-6200 Wiesbaden (DE)
(74) Vertreter: Beil, Hans Christoph, Dr.

(56) Entgegenhaltungen:
- AMERICAN REVIEW OF RESPIRATORY DISEASE, Band 137, nr. 4, Teil 2, April 1988, Seite 138, New York, US; C.M. LILLY et al.: "Pentoxifylline prevents hypotension and pulmonary injury caused by tumor necrosis factor"
- BIOCHEMICAL AND BIOPHYSICAL RESEARCH COMMUNICATIONS, Band 155, Nr. 3, September 1988, Seiten 1230-1236, New York, US; R.M. STRIETER et al.: "Cellular and molecular regulation of tumor necrosis factor-alpha production by pentoxifylline"
- NATURE, Band 330, Nr. 17, Dezember 1987, Seiten 662-664, London, GB; K.J. TRACEY et al.: "Anti-cachectin/TNF monoclonal antibodies prevent septic shock during lethal bacteraemia"

## Beschreibung

Es ist bekannt, daß unter der Bezeichnung Tumor Necrosis Factor (TNF) zwei zytotoxische Faktoren verstanden werden (TNF-α und TNF-β), die im wesentlichen von aktivierten Lymphozyten und Monozyten gebildet werden. Der TNF verdankt seine Bezeichnung einer Eigenschaft, die anfangs in der Fachwelt besondere Aufmerksamkeit fand: TNF wirkt bei bestimmten Tumoren in vivo nekrotisch. Erstmalig wurde diese Wirkung festgestellt, als man beobachtete, daß bakterielle Infektionen bei Krebserkrankungen zu einem Rückgang des Tumors führen können. Zunächst wurde angenommen, daß Lipopolysaccharide aus Zellwänden von Bakterien, die als Endotoxin bezeichnet werden, für den Rückgang des Tumors verantwortlich sind. Es konnte jedoch später gezeigt werden, daß das Endotoxin selbst keine direkte Antitumorwirkung hat, sondern seinerseits die Bildung eines Faktors induziert, der für die Tumornekrose verantwortlich ist. Dieser Faktor wurde dann TNF genannt. Weitere Untersuchungen haben später ergeben, daß TNF vor allem von Makrophagen gebildet wird. Durch die Stimulation von Lymphozyten in vitro konnte ebenfalls gezeigt werden, daß eine Substanz mit zytotoxischer Aktivität gebildet wird, die als Lymphotoxin bezeichnet wurde. Die enge Verwandtschaft von TNF und Lymphotoxin wurde erst kürzlich entdeckt, nachdem beide Proteine in reiner Form hergestellt werden, ihre Primärstruktur festgestellt und ihre cDNA's isoliert und exprimiert werden konnten. Dabei stellten sich erstaunliche Ähnlichkeiten in den beiden Proteinen und ihren Genen heraus. Starke Ähnlichkeiten zeigten sich auch in den biologischen Aktivitäten dieser beiden Proteine. Deshalb wurden sie als TNF-α und TNF-β bezeichnet, entsprechend ihrer Bildung aus Monozyten oder Lymphozyten.

Nachdem durch die Anwendung gentechnologischer Herstellungsmethoden größere Mengen von TNF zugänglich wurden, zeigte sich, daß TNF neben seiner Antitumorwirkung noch eine Fülle von weiteren Wirkungen hat. Sie sind im einzelnen beschrieben von Bharat B. Aggarwal in "Drugs of the Future" 12 (1987), Seite 891 ff. Insgesamt muß festgestellt werden, daß der TNF im Organismus eine wichtige Aufgabe im Immunsystem erfüllt, daß es jedoch bei bestimmten Infektionen, Erkrankungen oder Verletzungen zu einer Überproduktion von TNF kommen kann, die lebensbedrohend sein kann. Es ist deshalb auch schon vorgeschlagen worden, bei bestimmten Krankheitszuständen, die durch eine Überproduktion von TNF gekennzeichnet sind, einen TNF-Inhibitor einzusetzen. Hierfür wurden bereits gegen TNF gerichtete monoklonale Antikörper vorgeschlagen (Nature, Vol. 330, Seite 662 ff.). Es ist außerdem schon bekannt, daß nach vorheriger Verabreichung von Pentoxifyllin ein durch Endotoxin induzierter Schock vermieden werden kann (Clinical Hemorheology, Vol. 6, 1986, Seiten 455-467).

Die vorliegende Erfindung geht nun von der Überlegung aus, daß die Unverträglichkeit bestimmter Arzneimittel möglicherweise dadurch verursacht sein kann, daß nach ihrer Verabreichung im Organismus TNF in so großen Mengen freigesetzt wird, daß Nekrosen oder andere für eine Überproduktion von TNF charakteristische Nebenwirkungen auftreten. Diese Überlegung wurde durch Untersuchungen mit dem Antimykotikum Amphotericin B bestätigt, von dem bekannt ist, daß es nach parenteraler Verabreichung im Tierversuch zu einer 100 %igen Letalität führt, wenn bestimmte Wirkstoffmengen überschritten werden. Gibt man Amphotericin B jedoch zusammen mit einem TNF-Inhibitor, dann wird das sonst hoch toxische Amphotericin B ohne Nebenwirkungen vertragen.

Die Erfindung betrifft ein Kombinationspräparat, enthaltend Amphotericin B als Tumor Neurosis Factor (TNF)-freisetzende Substanz und ein Xanthinderivat als TNF-Inhibitor zur Behandlung von Nebenwirkungen, die durch Amphotericin B verursacht werden.

Bevorzugte Xanthinderivate stammen aus der Gruppe der folgenden Verbindungen:
1) Verbindungen der Formel I 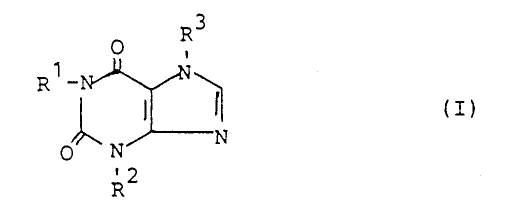 in der einer der Reste R¹ und R³ eine geradkettige Alkyl-, (ω-1)-Oxoalkyl- oder (ω-1)-Hydroxyalkylgruppe mit 3 bis 8 C-Atomen und die beiden anderen Reste R² und R³ oder R¹ und R² geradkettige oder verzweigte Alkylgruppen mit 1 bis 8 C-Atomen in der Position von R¹ und R³ und 1 bis 4 C-Atomen in der Position von R² darstellen, wobei die Summe der C-Atome dieser beiden Alkylsubstituenten höchsten 10 beträgt,
2) Verbindungen der Formel II 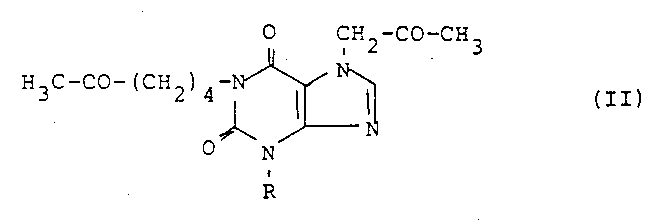 in der R für einen Alkylrest mit 1 bis 4 C-Atomen steht,
3) Verbindungen der Formel III 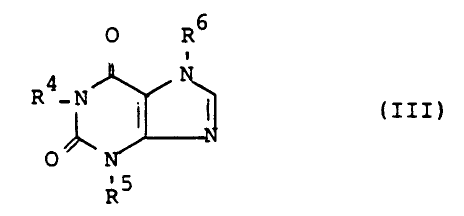 in der mindestens einer der Reste R⁴ und R⁶ eine tertiäre Hydroxyalkylgruppe der Formel 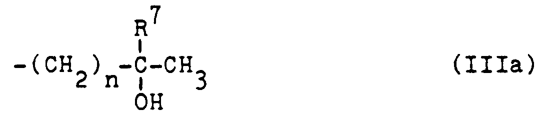 darstellt, wobei R⁷ eine Alkylgruppe mit bis zu 3 C-Atomen und n eine ganze Zahl von 2 bis 5 bedeuten, und - falls nur einer der Reste R⁴ oder R⁶ eine solche tertiäre Hydroxyalkylgruppe der Formel IIIa bedeutet - der andere Rest für ein Wasserstoffatom oder einen aliphatischen Kohlenwasserstoff-Rest R⁸ mit bis zu 6 C-Atomen steht, dessen Kohlenstoffkette von bis zu 2 Sauerstoffatomen unterbrochen oder mit einer Oxogruppe oder bis zu zwei Hydroxygruppen substituiert sein kann (wobei eine im Rest R⁸ befindliche Oxo- oder Hydroxygruppe vorzugsweise durch mindestens 2 C-Atome vom Stickstoff getrennt ist), und R⁵ eine Alkylgruppe mit 1 bis 4 C-Atomen darstellt,
4) Prodrug-Formen der Verbindungen der Formel I bis III und/oder
5) Metabolite der Verbindungen der Formeln I bis III.

Unter diesen Verbindungen sind wiederum jene der Formel I besonders bevorzugt, die in der Position von R¹ oder R³ eine Hexyl-, 5-Oxohexyl- oder 5-Hydroxyhexylgruppe tragen. Zu ihnen gehören insbesondere
1-Hexyl-3,7-dimethylxanthin,
1-(5-Hydroxyhexyl)-3,7-dimethylxanthin,
3,7-Dimethyl-1-(5-oxohexyl)-xanthin,
7-(5-Hydroxyhexyl)-1,3-dimethylxanthin,
1,3-Dimethyl-7-(5-oxohexyl)-xanthin,
1-(5-Hydroxyhexyl)-3-methyl-7-propylxanthin und
3-Methyl-1-(5-oxohexyl)-7-propylxanthin,
vor allem 3,7-Dimethyl-1-(5-oxohexyl)-xanthin (= Pentoxifyllin).

Besonders bevorzugte Verbindungen der Formel III sind solche Verbindungen, bei denen R⁵ für eine Methyl- oder Ethylgruppe steht. Gleichermaßen bevorzugt sind jene Verbindungen der Formel III, in denen nur einer der beiden Reste R⁴ oder R⁶ die vorstehend definierte tertiäre Hydroxyalkylgruppe darstellt. Weiterhin bevorzugt sind solche Verbindungen, bei denen R⁷ für eine Methylgruppe steht und n eine ganze Zahl von 3 bis 5 bedeutet, so daß der tertiäre Hydroxyalkylrest IIIa entweder [(ω-1)-Hydroxy-(ω-1)-methyl]-pentyl, -hexyl oder -heptyl darstellt, insbesondere solche, bei denen R⁵ Methyl oder Ethyl bedeutet.

Weiterhin sind diejenigen Verbindungen der Formel III besonders hervorzuheben, in denen R⁴ die tertiäre Hydroxyalkylgruppe darstellt und R⁶ für Alkyl, Hydroxyalkyl oder Alkoxyalkyl mit jeweils 1 bis 4 C-Atomen steht, wie etwa das 7-Ethoxymethyl-1-(5-hydroxy-5-methylhexyl)-3-methylxanthin.

Eine weitere Ausführungsform der Erfindung besteht darin, daß die Oxoalkylxanthine der Formel I und II bzw. die Hydroxyalkylxanthine der Formel I und III nicht per se, sondern in Prodrug-Form eingesetzt werden, aus der erst im Organismus die therapeutisch wirksamen Xanthinverbindungen mit den in Formeln I, II und III definierten Substituenten durch Biotransformation freigesetzt werden können. Hierfür kommen beispielsweise die acetalisierten Oxoalkylxanthine, in denen die Carbonylgruppen durch das Strukturelement der Formel IV 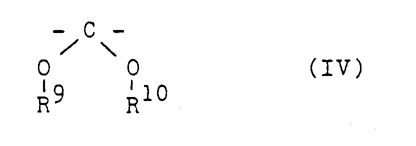
ersetzt sind, und die O-acylierten Hydroxyalkylxanthine mit dem Strukturelement der Formel (V)

R¹¹-CO-O- (V)

anstelle der Hydroxyfunktion in Frage, wobei R⁹ und R¹⁰ jeweils eine Alkylgruppe mit bis zu 4 C-Atomen oder zusammen eine Ethylene-, Trimethylen- oder Tetramethylengruppe darstellen und R¹¹ einen Alkylrest mit bis zu 4 C-Atomen oder gegebenenfalls substituiertes Phenyl oder Pyridyl bedeutet.

Das Gewichtsverhältnis der TNF-freisetzenden Substanz zum Xanthinderivat in den erfindungsgemäßen Kombinationspräparaten kann einen weiten Bereich überstreichen. Zu bevorzugen ist ein Gewichtsverhältnis von ca. 1:100 bis ca. 100:1, besonders bevorzugt von ca. 1:10 bis ca. 10:1.

Die erfindungsgemäßen Kombinationspräparate können auf unterschiedliche Weise verabreicht werden. Z.B. können sie intravenös, intramuskulär, intraperitoneal, subkutan oder oral verabreicht werden.

Die Herstellung der erfindungsgemäßen Kombinationspräparate erfolgt, indem Amphotericin B und mindestens ein Xanthinderivat gegebenenfalls mit weiteren Zusatz- und/oder Hilfsstoffen in eine geeignete Darreichungsform gebracht wird. Die Zusatz- oder Hilfsstoffe stammen aus der Gruppe der Trägermittel, Konservierungsstoffe und anderer üblicher Hilfsstoffe. Z.B. können für orale Darreichungsformen Hilfsstoffe wie Stärke, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente des Kombinationspräparats, ein Retardierungsmittel zuzusetzen, gegebenenfalls in Form von permeablen Membranen, wie z.B. solche auf Cellulose- oder Polystyrolharzbasis, oder Ionenaustauscher.

Es ist überraschend, daß die genannten Xanthinderivate, obwohl sie selbst nach wenigen Stunden den Organismus schon wieder verlassen haben, dennoch die unerwünschten Nebenwirkungen von TNF-freisetzenden Substanzen unterbinden können, selbst wenn diese nur über einen sehr langen Zeitraum aus dem Organismus ausgeschieden werden. So ist das Amphotericin B noch nach 12 Stunden im Organismus nachweisbar, während ein Xanthinderivat wie Pentoxifyllin schon nach 3 bis 4 Stunden aus dem Organismus wieder ausgeschieden wird. Trotzdem reicht die TNF-inhibierende Wirkung des Xanthinderivates aus.

Die TNF-inhibierende Wirkung von Xanthinderivaten konnte in folgender Weise experimentell nachgewiesen werden:
Amphotericin B ist eine Substanz, bei der gezeigt werden konnte, daß die hochtoxischen Nebenwirkungen auf die Freisetzung von TNF zurückzuführen sind. Nachweisen läßt sich die Freisetzung von TNF im Mäuseserum durch ein spezifisches ELISA-Verfahren mit murinem monoklonalen Anti-TNF. Gibt man Mäusen Amphotericin B in Mengen von 100 mg/kg oder höher intraperitoneal, dann wird 100 %ige Mortalität festgestellt. Die in der beiliegenden Tabelle zusammengestellten Versuchsergebnisse zeigen, daß die toxische Wirkung von Amphotericin B auch nicht durch Dexamethason überwunden werden kann. Verabreicht man jedoch das Amphotericin B zusammen mit Pentoxifyllin, dann überleben bei Verabreichung von 100 mg/kg Pentoxifyllin alle Mäuse, die 100 oder 150 mg/kg Amphotericin B erhalten haben. Erst bei der Verabreichung einer übergroßen Dosis von 200 mg/kg Amphotericin B kann eine Gabe von 100 mg/kg Pentoxifyllin die Nebenwirkungen nicht mehr neutralisieren.

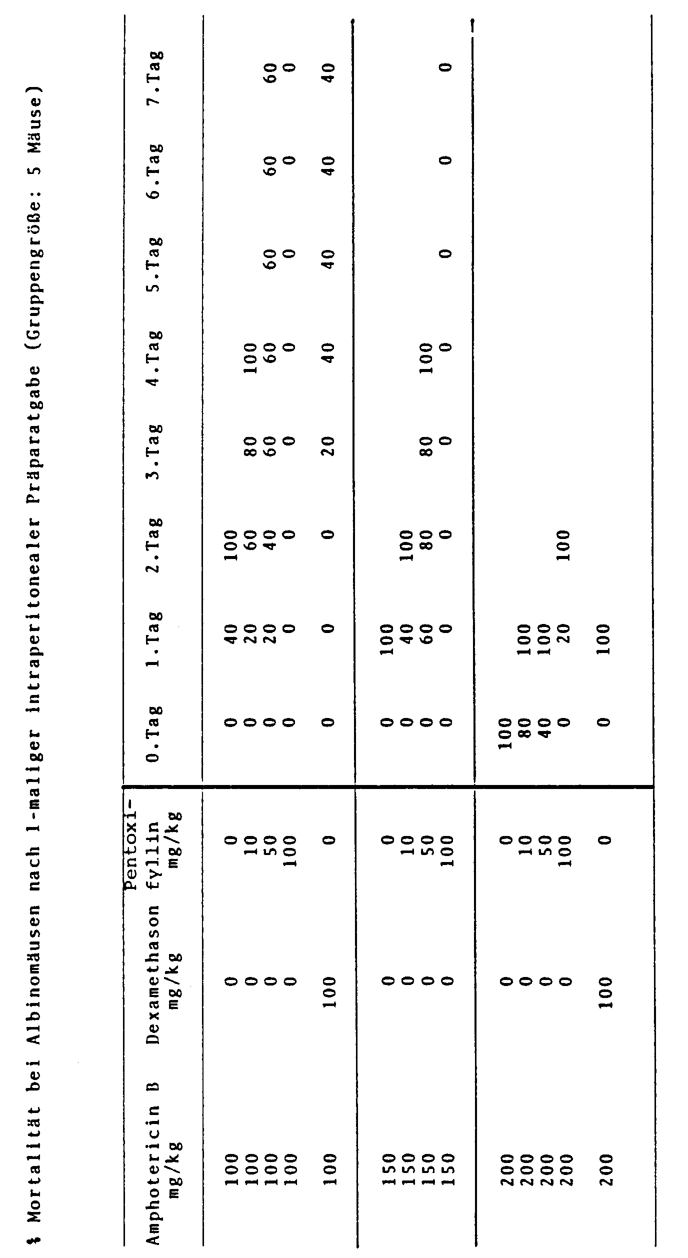

## Patentansprüche (Patentansprüche für folgende(n) Vertragsstaat(en): AT, BE, CH, DE, FR, GB, IT, LI, LU, NL, SE)

1. Kombinationspräparat, enthaltend Amphotericin B als Tumor Necrosis Factor (TNF)-freisetzende Substanz und ein Xanthinderivat als TNF-Inhibitor zur Behandlung von Nebenwirkungen, die durch Amphotericin B verursacht werden.

2. Kombinationspräparat nach Anspruch 1, dadurch gekennzeichnet, daß es als TNF-Inhibitor Pentoxifyllin und/oder Propentofyllin enthält.

3. Verwendung des Kombinationspräparates nach Anspruch 1 zur Herstellung eines Arzneimittels zur Behandlung von Nebenwirkungen, die durch Amphotericin B verursacht werden.

4. Verwendung nach Anspruch 3, dadurch gekennzeichnet, daß als Xanthinderivat Pentoxifyllin und/oder Propentofyllin eingesetzt wird.

## Patentansprüche (Patentansprüche für folgende(n) Vertragsstaat(en): ES, GR)

1. Verfahren zur Herstellung eines Kombinationspräparates zur Behandlung von Nebenwirkungen, die durch Amphotericin B verursacht werden, dadurch gekennzeichnet, daß Amphotericin B zusammen mit einem Xanthinderivat als TNF-Inhibitor gegebenenfalls unter Zusatz von pharmakologisch verträglichen Hilfs- und/oder Trägerstoffen in eine geeignete Darreichungsform gebracht wird.

2. Verfahren gemäß Anspruch 1, dadurch gekennzeichnet, daß als TNF-Inhibitor Pentoxifyllin und/oder Propentofyllin eingesetzt wird.

3. Verwendung von Amphotericin B und einem Xanthinderivat als TNF-Inhibitor zur Herstellung eines Arzneimittels zur Behandlung von Nebenwirkungen, die durch Amphotericin B verursacht werden.

4. Verwendung gemäß Anspruch 3, dadurch gekennzeichnet, daß als Xanthinderivat Pentoxifyllin und/oder Propentofyllin eingesetzt wird.

## Claims (Claims for the following Contracting State(s): AT, BE, CH, DE, FR, GB, IT, LI, LU, NL, SE)

1. Combination preparation, containing Amphotericin B as Tumour Necrosis Factor (TNF)-liberating substance and a xanthine derivative as TNF inhibitor, for the treatment of side effects caused by Amphotericin B.

2. Combination preparation according to claim 1, characterised in that it contains pentoxifylline and/or propentofylline as TNF inhibitor.

3. Use of the combination preparation according to claim 1 for the preparation of a medicine for the treatment of side effects caused by Amphotericin B.

4. Use according to claim 3, characterised in that pentoxifylline and/or propentofylline is used as xanthine derivative.

## Claims (Claims for the following Contracting State(s): ES, GR)

1. Method for the preparation of a combination preparation for the treatment of side effects caused by Amphotericin B, characterised in that Amphotericin B together with a xanthine derivative as TNF inhibitor, optionally with the addition of pharmacologically compatible auxiliary substances and/or carriers, is brought into a form suitable for administration.

2. Method according to claim 1, characterised in that pentoxifylline and/or propentofylline is used as TNF inhibitor.

3. Use of Amphotericin B and of a xanthine derivative as TNF inhibitor for the preparation of a medicine for the treatment of side effects caused by Amphotericin B.

4. Use according to claim 3, characterised in that pentoxifylline and/or propentofylline is used as xanthine derivative.

## Revendications (Revendications pour l'(les) Etat(s) contractant(s) suivant(s): AT, BE, CH, DE, FR, GB, IT, LI, LU, NL, SE)

1. Préparation combinée contenant de l'amphotéricine B comme substance libérant le facteur de nécrose de tumeur (TNF) et un dérivé de xanthine comme inhibiteur de TNF pour traiter les effets secondaires qui sont provoqués par l'amphotéricine B.

2. Préparation combinée selon la revendication 1, caractérisée en ce qu'elle contient comme inhibiteur de TNF de la pentoxyfylline et/ou de la propentofylline.

3. Utilisation de la préparation combinée selon la revendication 1 pour préparer un médicament de traitement des effets secondaires qui sont provoqués par l'amphotéricine B.

4. Utilisation selon la revendication 3, caractérisée en ce qu'on utilise comme dérivé de xanthine de la pentoxyfylline et/ou de la propentofylline.

## Revendications (Revendications pour l'(les) Etat(s) contractant(s) suivant(s): ES, GR)

1. Procédé de préparation d'une préparation combinée pour traiter les effets secondaires qui sont provoqués par l'amphotéricine B, caractérisé en ce qu'on met sous une forme d'administration appropriée l'amphotéricine B en présence d'un dérivé de xanthine comme inhibiteur de TNF le cas échéant en ajoutant des additifs et/ou véhicules pharmaceutiquement compatibles sous une forme d'administration appropriée.

2. Procédé selon la revendication 1, caractérisé en ce qu'on utilise comme inhibiteur de TNF de la pentoxifylline et/ou de la propentofylline.

3. Utilisation d'amphotéricine B et d'un dérivé de xanthine comme inhibiteur de TNF pour produire un médicament pour traiter les effets secondaires qui sont provoqués par l'amphotéricine B.

4. Utilisation selon la revendication 3, caractérisée en ce qu'on utilise comme dérivé de xanthine de la pentoxifylline et/ou de la propentofylline.
